# EUROPEAN PATENT APPLICATION

(11) **EP 3 831 942 A1**
(43) Date of publication of application: **09.06.2021**
(21) Application number: 19843279.1
(22) Date of filing: 02.08.2019
(51) Int. Cl.: C12N 15/11, A61K 31/7088, A61K 31/7115, A61K 48/00, A61P 25/00, A61P 25/14, A61P 25/16, A61P 25/28, A61P 43/00

(54) **TDP-43 AGGREGATION INHIBITOR**

(30) Priority: 03.08.2018 JP 2018146891
(71) Applicant: NATIONAL UNIVERSITY CORPORATION TOKYO MEDICAL AND DENTAL UNIVERSITY, Tokyo Bunkyo-ku 1138510 (JP)
(72) Inventor: YOKOTA, Takanori, Tokyo 113-8510 (JP); SAKAUE, Fumika, Tokyo 113-8510 (JP)
(74) Representative: J A Kemp LLP
(86) International application number: PCT/JP2019/030407
(87) International publication number: WO 2020/027311

(57) **Abstract**

This invention provides a nucleic acid that can attenuate toxicity caused by abnormal accumulation of RNA-binding proteins in cells and can be used for prevention or treatment of neurodegenerative diseases and an agent for prevention or treatment of neurodegenerative diseases containing such nucleic acid. Such nucleic acid comprises a repeat RNA consisting of nucleotides represented by the formula: (U(Me)GX₁X₂X₃...Xₘ)ₙ, wherein U(Me) is 2'-O-Me-modified; X₁, X_{2,} X₃, Xₘ may or may not be present, each independently represent A, C, G, or U in the presence thereof, and may be the same or different; m is 1 to 10; G, X₁, X₂, X₃, ...Xₘ may or may not be each independently 2'-O-Me-modified; and n is 4 to 15. The agent for prevention or treatment of neurodegenerative diseases induced by abnormal accumulation of TDP-43 in cells comprises such nucleic acid as an active ingredient.

SEQUENCE LISTING

<110>  National University Coporation Tokyo Medical and Dental University

<120>  An inhibitor of TDP-43 aggregation

<130>  PH-8056-PCT

<150>  JP 2018-146891
<151>  2018-08-03

<160>  2

<170>  PatentIn version 3.5

<210>  1
<211>  34
<212>  RNA
<213>  Artificial

<220>
<223>  CLIP34

<400>  1
gagagagcgc gugcagagac uugguggugc auaa                                34


<210>  2
<211>  30
<212>  RNA
<213>  Artificial

<220>
<223>  tb14

<400>  2
gaagugggau gugggaagag ggauguugug                                     30

## Description

### [Technical Field]

The present invention relates to a nucleic acid that can be used for prevention or treatment of neurodegenerative diseases such as amyotrophic lateral sclerosis (ALS).

### [Background Art]

Amyotrophic lateral sclerosis (ALS) is an intractable neurological disease caused by selective denaturation of upper and lower motor neurons leading to muscular weakness of the entire body. In Japan, the prevalence rate of ALS is 7 to 11 persons per 100,000 persons, and the annual incidence rate is approximately 1 to 2.5 persons per 100,000 persons. Primary symptoms are amyotrophia and muscular weakness. As the disease progresses, limb and trunk dysfunctions, gait disturbance, and cranial nerve disorders, such as articulation disorder and swallowing difficulty, occur, which lead to respiratory disturbance caused by respiratory muscle paralysis in the end. Disease progression is relatively fast, and, in general, patients often die in 2 to 5 years without the use of artificial respirators. As a therapeutic agent, a drug that can deter ALS progression (Riluzole; tradename: Rilutek®) has been used; although satisfactory therapeutic effects are not yet achieved.

In recent years, RNA-binding proteins with common functions and structures, such as TDP-43 and FUS/TLS, were found to be deeply involved in the pathological conditions of ALS (see Non-Patent Literature 1). In particular, TDP-43-positive inclusion bodies observed in degenerated neurons are pathological images that are observed in common among a majority of ALS cases as well as sporadic ALS. Specifically, TDP-43 plays a key role in development and pathological conditions of ALS. At present, research on ALS has been conducted from the viewpoints of toxicity exerted by formation of RNA-binding protein aggregates in addition to abnormality in the control of functions and targets that are common among the RNA-binding proteins.

Research on amyotrophic lateral sclerosis had advanced to a significant extent as a result of finding of TDP-43 accumulation in degenerated neuronal cytoplasmic inclusion bodies. One of the pathological findings is as follows. That is, approximately 90% of TDP-43 is essentially nuclear-localized; however, substantially all TDP-43 is accumulated in degenerated neuronal cytoplasmic inclusion bodies and eliminated from the nuclei. In the degenerated neurons, accordingly, physiological functions of TDP-43 exerted in nuclei should have been lost. From the viewpoints of acquisition of toxicity such that the TDP-43 aggregate constituting the inclusion body exerts toxicity, which leads to cell death, and TDP-43 dysfunctions, research on the pathological conditions had advanced. In addition, FUS/TLS that had been physiologically nuclear-localized is found to be excessively retained in the cytoplasm of neuron cells of ALS patients and show abnormal localization (Non-Patent Literature 2).

Research that had been conducted in the past demonstrates that transgenic mice overexpressing wild-type human TDP-43 show amyotrophic lateral sclerosis-like symptoms because of degenerated motor neurons. Also, transgenic mice expressing mutant TDP-43, which was identified in hereditary amyotrophic lateral sclerosis, show amyotrophic lateral sclerosis-like symptoms. Such finding indicates that specific neurons such as motor neurons are particularly vulnerable to excessive TDP-43, regardless of the occurrence of TDP-43 mutation. In addition, excessive TDP-43 is reported to exert toxicity in the form of the RNA-binding ability of the RNA recognition motif (RRM) (Non-Patent Literature 3 and Non-Patent Literature 4).

The RNA sequence that attenuates toxicity of TDP-43 is reported (Patent Literature 1).

### [Prior Art Literatures]

### [Patent Literatures]

[Patent Literature 1] WO 2016/088797

### [Non-Patent Literatures]

[Non-Patent Literature 1] Clotilde Lagier-Tourenne et al., Nature Neuroscience 15, 1488-1497, 2012
[Non-Patent Literature 2] T. J. Kwiatkowski Jr., D. A. Bosco et al., Science, vol. 323, 1205-1208, 2009
[Non-Patent Literature 3] Ryoko Ihara, Koji Matsukawa et al., Hum. Mol. Genet., 2013, 22 (22): 4474-4484.
[Non-Patent Literature 4] J. Gavin Daiglel, Nicholas A et al., Hum. Mol. Genet., 2013, 22 (6): 1193-1205

### [Summary of the Invention]

### [Objects to Be Attained by the Invention]

The present invention provides a nucleic acid that can attenuate toxicity caused by abnormal accumulation of TDP-43 (transactivation responsive region (TAR) DNA-binding protein 43), which is an RNA-binding protein causing neurodegenerative diseases (e.g., ALS) in cells, and excessive RNA binding and that can be used for prevention or treatment of neurodegenerative diseases (e.g., ALS).

### [Means for Attaining the Objects]

The present inventors discovered that (UGGAA)ₙ repeat RNA could be used as a nucleic acid medicine that would first suppress TDP-43 aggregation to attenuate toxicity of TDP-43 and improve ALS symptoms (WO 2016/088797).

In addition, the present inventors searched for nucleotide sequences that would suppress TDP-43 aggregation, compared a repeat sequence of a nucleotide sequence starting from UG with another repeat sequence, and found that the repeat sequence of a nucleotide sequence starting from UG would exert more satisfactory effects. Also, the present inventors performed chemical modification, such as 2'-O-methylation, on an RNA ribose and examined as to whether or not the effects of suppressing TDP-43 aggregation would be enhanced.

As a result, they discovered that 2'-O-methylation of at least the first U would enhance the effects of suppressing TDP-43 aggregation to a significant extent. This has led to the completion of the present invention.

Specifically, the present invention is as follows.
[1] A natural or artificial nucleic acid that binds to an RNA recognition motif (RRM).
[2] The nucleic acid according to [1], which binds to an RNA recognition motif (RRM) of TDP-43.
[3] The nucleic acid according to [1] or [2], which comprises a repeat RNA consisting of nucleotides represented by the formula: U(Mod)GX₁X₂X₃...Xₘ)ₙ, wherein U(Mod) represents a U derivative having an oxygen atom at the ribose 2' position; X₁, X₂, X₃, ...Xₘ may or may not be present, each independently represent A, C, G, or U in the presence thereof, and may be the same or different; m is 1 to 10; G, X₁, X₂, X₃, ...Xₘ may or may not each independently represent a derivative having an oxygen atom at the 2' position; and n is 4 to 15.
[4] The nucleic acid according to [3], wherein U(Mod) represents 2'-O-methylated or 2'-O-methoxyethylated (MOE) U.
[5] The nucleic acid according to [4], which comprises a repeat RNA consisting of nucleotides represented by the formula: U(Me)GX₁X₂X₃...Xₘ)ₙ, wherein U(Me) is 2'-O-Me-modified; X₁, X₂, X₃, ...Xₘ may or may not be present, each independently represent A, C, G, or U in the presence thereof, and may be the same or different; m is 1 to 10; G, X₁, X_{2,} X_{3'} , ...Xₘ may or may not be each independently 2'-O-Me-modified; and n is 4 to 15.
[6] The nucleic acid according to [5], which comprises a repeat RNA consisting of nucleotides represented by the formula: (U(Me)G)ₙ or (U(Me)G(Me))ₙ, wherein U(Me) and G(Me) are each independently 2'-O-Me-modified; and n is 7 to 30.
[7] The nucleic acid according to [5], which comprises a repeat RNA consisting of nucleotides represented by the formula: (U(Me)G)₁ or (U(Me)G(Me))₁₂, wherein U(Me) and G(Me) are each independently 2'-O-Me-modified.
[8] The nucleic acid according to [5], which comprises a repeat RNA consisting of nucleotides represented by the formula: (U(Me)G(Me)G(Me)A (Me)A (Me))₅₋₁₀, wherein U(Me), G(Me), and A (Me) are 2'-O-Me-modified.
[9] The nucleic acid according to [4], which comprises a repeat RNA consisting of nucleotides represented by the formula: (U(MOE)GX₁X₂X₃...Xₘ)ₙ, wherein U(MOE) is 2'-O-methoxyethylated; X₁, X_{2,} X₃, Xₘ may or may not be present, each independently represent A, C, G, or U in the presence thereof, and may be the same or different; m is 1 to 10; G, X₁, X₂, X₃, ...Xₘ may or may not be each independently 2'-O-methoxyethylated; and n is 4 to 15.
[10] The nucleic acid according to [9], which comprises a repeat RNA consisting of nucleotides represented by the formula: (U(MOE)G)ₙ or (U(MOE)G(MOE))ₙ, wherein U(MOE) and G(MOE) are 2'-O-methoxyethylated; and n is 7 to 30.
[11] The nucleic acid according to [9], which comprises a repeat RNA consisting of nucleotides represented by the formula: (U(MOE)G)₁₂ or (U(MOE)G(MOE))₁₂, wherein U(MOE) and G(MOE) are 2'-O-methoxyethylated (MOE).
[12] The nucleic acid according to [9], which comprises a repeat RNA consisting of nucleotides represented by the formula: (U(MOE)G(MOE)G(MOE)A (MOE)A (MOE))₅₋₁₀, wherein U(MOE), G(MOE), and A (MOE) are 2'-O-methoxyethylated.
[13] The nucleic acid according to any of [3] to [12], wherein at least 1 RNA in the repeat RNA is substituted with DNA.
[14] The nucleic acid according to any of [1] to [4], which comprises at least 5 dinucleotide units represented by the formula: (U(Mod)G) or (U(Mod)G(Mod)), wherein U(Mod) and G(Mod) each independently represent 2'-O-methylated or 2'-O-methoxyethylated U and G.
[15] The nucleic acid according to any of [1] to [14], wherein the total number of nucleotides is 10 to 50.
[16] A pharmaceutical composition comprising, as an active ingredient, the nucleic acid according to any of [1] to [15].
[17] An agent for prevention or treatment of a neurodegenerative disease induced by abnormal accumulation of TDP-43 in cells comprising, as an active ingredient, the nucleic acid according to any of [1] to [15].
[18] The agent for prevention or treatment of a neurodegenerative disease according to [17], wherein the nucleic acid suppresses abnormal accumulation of TDP-43 in cells.
[19] The agent for prevention or treatment of a neurodegenerative disease according to [17] or [18], wherein the neurodegenerative disease is TDP-43 proteinopathy.
[20] The agent for prevention or treatment of a neurodegenerative disease according to any of [17] to [19], wherein the neurodegenerative disease is selected from the group consisting of amyotrophic lateral sclerosis (ALS), which is sporadic ALS or familial ALS, frontotemporal lobar degeneration (FTLD), Alzheimer's disease, Lewy body dementia, Huntington's disease, Parkinson's disease, argyrophilic grain dementia (grain disease), Perry syndrome, progressive supranuclear palsy, corticobasal degeneration, and Pick's disease.
[21] The agent for prevention or treatment of a neurodegenerative disease according to [20], wherein the neurodegenerative disease is amyotrophic lateral sclerosis (ALS), which is sporadic ALS or familial ALS, or frontotemporal lobar degeneration (FTLD).

This description contains part or all of the content as disclosed in the description and/or drawings of Japanese Patent Application No. 2018-146891, based on which the present application claims a priority.

### [Effects of the Invention]

A repeat RNA, which comprises the "n" number of a repeat sequence of RNA consisting of 2 to 10 nucleotides starting from 2 nucleotides; i.e., UG, with at least the first U being modified (e.g., 2'-O-Me-modified (2'-O-methylated : 2'-o-methylation) or MOE-modified), is capable of attenuating cytotoxicity resulting from abnormal accumulation or abnormal aggregation of TDP-43 in cells caused by abnormal expression of RNA-binding proteins (e.g., TDP-43) and excessive binding to RNA. Abnormal accumulation of RNA-binding proteins in cells caused by abnormal expression thereof induces neurodegenerative diseases, such as amyotrophic lateral sclerosis (ALS), which is sporadic ALS or familial ALS, and frontotemporal lobar degeneration (FTLD). Accordingly, such repeat RNA can be used for prevention or treatment of neurodegenerative diseases resulting from abnormal accumulation of RNA-binding proteins in cells.

### [Brief Description of the Drawings]

Fig. 1 shows a structure of a 2'-O-Me-modified nucleotide.
Fig. 2 shows a method for evaluating the inhibitory effects of synthetic nucleic acids on aggregation.
Fig. 3 shows the inhibitory effects of TDP-43-binding RNA (24- to 48-mers) on aggregation.
Fig. 4 shows the results of searching for chemical modification that enhances the effects of RNA of the (UG)₁₂ sequence.
Fig. 5 shows the effects of 2'-O-Me-modification on RNA of the (UG)₁₂ sequence.
Fig. 6 shows the inhibitory effects on aggregation depending on the repeat number of (U(Me)G(Me))n.
Fig. 7 shows the influence of 2'-O-Me-modification on the inhibitory effects on aggregation of RNA of the (UGGAA)ₙ sequence.
Fig. 8 shows stability of (UG)₁₂-2'-OMe in the cerebrospinal fluid (CSF).
Fig. 9 shows the results of evaluation of stability of (UG)₁₂-2'-OMe via quantitative real-time PCR.
Fig. 10 shows the results of detection of the administered (UG)₁₂-2'-OMe on the other side (the right brain).
Fig. 11 shows the results of co-staining of (UG)₁₂-2'-OMe and neural cells/glial cells.
Fig. 12 shows the inhibitory effects on TDP-43 aggregation achieved by administration of (UG)₁₂-2'-OMe.
Fig. 13 shows the effects of motor function improvement achieved by administration of (UG)₁₂-2'-OMe.
Fig. 14 shows suppression of body weight loss achieved by administration of (UG)₁₂-2'-OMe.
Fig. 15 shows a prolonged survival time achieved by administration of (UG)₁₂-2'-OMe.

### [Embodiments of the Invention]

Hereafter, the present invention is described in detail.

The nucleic acid of the present invention is a natural or artificial nucleic acid that binds to an RNA recognition motif (RRM), and such nucleic acid preferably binds to an RNA recognition motif (RRM) of TDP-43. More specifically, the nucleic acid of the present invention binds to TDP-43 (transactivation responsive region (TAR) DNA-binding protein 43), which is an RNA-binding protein causing ALS, and inhibits toxicity, accumulation, and aggregation of TDP-43.

The nucleic acid is preferably an RNA, which is a repeat RNA comprising the "n" number of a repeat sequence of RNA consisting of 2 to 10 nucleotides starting from 2 nucleotides; i.e., UG. The total number of nucleotides constituting such repeat RNA can be 10 to 50. At least the first U is a U derivative having an oxygen atom at the ribose 2' position. The U derivative having an oxygen atom at the ribose 2' position is indicated as U(Mod). Other nucleotides may or may not be derivatives each having an oxygen atom at the 2' position, and the second G is preferably a derivative having an oxygen atom at the 2' position. All the nucleotides are preferably derivatives each having an oxygen atom at the 2' position.

An example of a derivative having an oxygen atom at the ribose 2' position is a 2'-O-methylated or 2'-O-methoxyethylated (MOE) derivative.

The total number of nucleotides constituting the nucleic acid of the present invention can be 10 to 50. For example, the total number of nucleotides can be 18 to 48, and, more specifically, it can be 18, 20, 22, 24, 26, 28, 30, 32, 34, 36, 38, 40, 42, 44, 46, or 48.

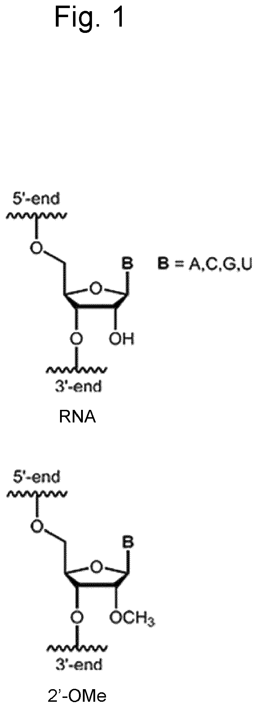

Specifically, the nucleic acid is preferably an RNA, which is a repeat RNA comprising the "n" number of a repeat sequence of RNA consisting of 2 to 10 nucleotides starting from 2 nucleotides; i.e., UG. The total number of nucleotides constituting such repeat RNA can be 10 to 50. At least the first U is subjected to 2'-O-Me-modification (2'-O-methylation). The term "2'-O-methylation (Me)" is also referred to as 2'-O-Me-modification. Other nucleotides may or may not be 2'-O-Me-modified, and the second G is preferably 2'-O-Me-modified. All the nucleotides are preferably 2'-O-Me-modified. When a 2'-O-Me-modified nucleotide is U, in the present invention, such nucleotide is indicated as U(Me) or U(2'OMe). Fig. 1 shows a structure of a 2'-O-Me-modified nucleotide. The total number of nucleotides constituting the nucleic acid of the present invention can be 10 to 50. For example, the total number of nucleotides can be 18 to 48, and, more specifically, it can be 18, 20, 22, 24, 26, 28, 30, 32, 34, 36, 38, 40, 42, 44, 46, or 48.

The nucleic acid is preferably an RNA, which is a repeat RNA comprising the "n" number of a repeat sequence of RNA consisting of 2 to 10 nucleotides starting from 2 nucleotides; i.e., UG. The total number of nucleotides constituting such repeat RNA can be 10 to 50. At least the first U is 2'-O-methoxyethylated. Other nucleotides may or may not be 2'-O-methoxyethylated, and the second G is preferably 2'-O-methoxyethylated. All the nucleotides are preferably 2'-O-methoxyethylated. When a 2'-O-methoxyethylated nucleotide is U, in the present invention, such nucleotide is indicated as U(MOE). The term "2'-O-methoxyethylation" is also referred to as 2'-O-methoxyethyl modification.

In the nucleic acid, both the 2'-O-methylated nucleic acid and the 2'-O-methoxyethylated (MOE) nucleic acid may be present.

Hereafter, the 2'-O-Me-modified nucleotide is described. As described above, a nucleotide may be a derivative of a nucleotide comprising an oxygen atom at the 2' position, and examples of derivatives of nucleotides each comprising an oxygen atom at the 2' position include a 2'-O-Me-modified nucleotide and a 2'-O-methoxyethylated nucleotide. In the description below, accordingly, "U(Me)" can be substituted with "U(Mod)" or "U(MOE)," "G(Me)" can be substituted with "G(Mod)" or "G(MOE)," "A (Me)" can be substituted with "A (Mod)" or "A (MOE)," and "C (Me)" can be substituted with "C (Mod)" or "C (MOE)."

The total number of nucleotides constituting the nucleic acid of the present invention can be 10 to 50. For example, the total number of nucleotides can be 18 to 48, and, more specifically, it can be 18, 20, 22, 24, 26, 28, 30, 32, 34, 36, 38, 40, 42, 44, 46, or 48.

The repeat RNA is represented by a formula: (U(Me)GX₁X₂X₃...Xₘ)ₙ. In the formula, X₁, X₂, X₃, ...Xₘ may or may not be present, each independently represent A, C, G, or U in the presence thereof, and may be the same or different. m is 1 to 10 and is preferably 5. In the formula above, specifically, X₁, X₂, X₃, ...Xₘ each comprise one to "m" number (e.g., 5) of nucleotides, and each nucleotide independently represents A, C, G, or U. Each of G, X₁, X₂, X₃, ...Xₘ may or may not be 2'-O-Me-modified. G is preferably 2'-O-Me-modified. All the nucleotides are preferably 2'-O-Me-modified. "n" indicates the number of repeats, it is 2 to 30, and it is preferably 5 to 25 or 4 to 15.

In the formulae above, (U(Me)G)ₙ or (U(Me)G(Me))ₙ is preferable. In this case, "n" is 7 to 30, and preferably 7 to 25. An example of U(Me)GX₁X₂X₃...Xₘ is U(Me)GGAA.

Specific examples include the sequences below.
(U(Me)G)₅₋₁₅
(U(Me)G)₁₂
(U(Me)G(Me))₅₋₁₅
(U(Me)G(Me))₁₂
(U(Me)GGAA)₅₋₁₀
(U(Me)GGAA)₈
(U(Me)G(Me)GAA)₅₋₁₀
(U(Me)G(Me)GAA)₈
(U(Me)G(Me)G(Me)AA)₅₋₁₀
(U(Me)G(Me)G(Me)AA)₈
(U(Me)G(Me)G(Me)A (Me)A)₅₋₁₀
(U(Me)G(Me)G(Me)A (Me)A)₈
(U(Me)G(Me)G(Me)A (Me)A (Me))₅₋₁₀
(U(Me)G(Me)G(Me)A (Me)A (Me))₈
(U(Me)GG(Me)AA)₅₋₁₀
(U(Me)GG(Me)AA)₈
(U(Me)GGA (Me)A)₅₋₁₀
(U(Me)GGA (Me)A)₈
(U(Me)GGAA (Me))₅₋₁₀
(U(Me)GGAAA (Me))₈
(U(Me)G(Me)GA (Me)A)₅₋₁₀
(U(Me)G(Me)GA (Me)A)₈
(U(Me)G(Me)GAA (Me))₋₁₀
(U(Me)G(Me)GAA (Me))₈
(U(Me)GG(Me)A (Me)A)₅₋₁₀
(U(Me)GG(Me)A (Me)A)₈
(U(Me)GG(Me)AA (Me))₋₁₀
(U(Me)GG(Me)AA (Me))₈
(U(Me)GGA (Me)A (Me))₅₋₁₀
(U(Me)GGA (Me)A (Me))₈
(U(Me)GG(Me)A (Me)A (Me))₅₋₁₀
(U(Me)GG(Me)A (Me)A (Me))₈
(U(Me)G(Me)GA (Me)A (Me))₅₋₁₀
(U(Me)G(Me)GA (Me)A (Me))₈
(U(Me)G(Me)G(Me)AA (Me))₅₋₁₀
(U(Me)G(Me)G(Me)AA (Me))₈

In the present invention, such RNA repeats are referred to as short-chain repeat RNAs.

In the nucleic acid, both the 2'-O-methylated nucleic acid and the 2'-O-methoxyethylated (MOE) nucleic acid may be present. In the formulae above, accordingly, each "(Me)" can be independently substituted with "(MOE)."

The present invention encompasses a nucleic acid comprising at least 5 dinucleotide units represented by the formula; for example, (U(Mod)G) or (U(Mod)G(Mod)), wherein U(Mod) and G(Mod) each independently represent 2'-O-methylated or 2'-O-methoxyethylated U and G. The total number of nucleotides constituting the nucleic acid of the present invention can be 10 to 50. For example, the total number of nucleotides can be 18 to 48, and, more specifically, it can be 18, 20, 22, 24, 26, 28, 30, 32, 34, 36, 38, 40, 42, 44, 46, or 48.

In the repeat RNA of the present invention, as described above, it is necessary that at least the first U in the nucleotide sequence serving as a repeat unit is 2'-O-Me-modified. When a nucleotide sequence is partially 2'-O-Me-modified, the sequence is deduced to acquire enhanced hydrophobic properties and a conformation that can easily bind to TDP-43.

The nucleic acid of the present invention comprises the repeat RNA described above. Specifically, other nucleic acids may be added to the 5' side and the 3' side of the repeat RNA described above. The types and the number of nucleotides constituting the nucleic acids upstream and downstream of the repeat sequence are not particularly limited. As long as the repeat sequence is present in the nucleic acid, such repeat sequence binds to the RNA-binding protein and exerts its effects. In other words, the nucleic acid of the present invention can be essentially composed of the repeat RNA described above.

In addition, the nucleic acid of the present invention encompasses a nucleic acid resulting from substitution of an RNA region of the short-chain repeat RNA with DNA. It is sufficient if at least 1 RNA region of the short-chain repeat RNA is substituted with DNA, and at least 10%, at least 20%, at least 30%, at least 40%, at least 50%, at least 60%, at least 70%, at least 80%, at least 90%, or all of RNAs may be substituted with DNAs. In the nucleic acid of the present invention, in addition, some or all of nucleoside-nucleoside bonds may be modified. For example, some or all of nucleoside-nucleoside bonds may be phosphorothioated.

The nucleic acid of the present invention binds to an RNA-binding protein. An RNA-binding protein binds to RNA and plays a key role in RNA metabolizing functions, such as RNA splicing, miRNA synthesis, and RNA transportation. When RNA-binding proteins are abnormally accumulated in cells and bind to a target group of RNAs, such RNA-binding proteins adversely affect the RNA metabolism functions causing abnormal splicing, abnormal transportation, and the like and exert toxicity on the cells. RNA-binding proteins also exert toxicity upon abnormal aggregation in the cytoplasm. When such RNA-binding proteins constitute an aggregate in a neuron, such RNA-binding proteins exert toxicity on the neuron, and various neurodegenerative diseases are induced. The nucleic acid of the present invention binds to an RNA recognition motif (RRM) of the RNA-binding protein abnormally expressed in the cytoplasm and blocks the sequence. Thus, the nucleic acid of the present invention functions as an absorber or a decoy that competes with the RNA-binding protein for RNA binding and attenuates toxicity of the RNA-binding protein.

Accordingly, the nucleic acid of the present invention can be used for prevention or treatment of diseases caused by abnormal accumulation of RNA-binding proteins in cells or abnormal aggregation thereof in the cytoplasm.

The nucleic acid of the present invention can be used for prevention or treatment of neurodegenerative diseases caused by aggregation of TDP-43. TDP-43 is a protein of the hnRNP family, and it belongs to a group of RNA chaperons that binds to RNA and plays a key role in RNA metabolizing functions, such as RNA splicing, miRNA synthesis, and RNA transportation. TDP-43 has an RNA recognition motif (an RNA-binding motif (RBM)) or a glycine-rich domain, and the nucleic acid of the present invention binds to an RNA recognition motif. The TDP-43 familial mutation mainly occurs in the glycine-rich domain, and such mutation leads to enhanced TDP-43 protein aggregation. When TDP-43 is overexpressed in animal models such as mice or fruit flies, TDP-43 is aggregated in the cytoplams, and TDP-43 exerts toxicity. In the neurons, the nerve is degenerated, and neurodegenerative diseases are induced. When the nucleic acid of the present invention binds to TDP-43, for example, accumulation or aggregation of TDP-43 occurring in cells can be suppressed, and TDP-43 cytotoxicity can be attenuated.

Examples of diseases resulting from abnormal aggregation of TDP-43 in the cytoplasm and formation of inclusion bodies in cells include neurodegenerative diseases. The term "neurodegenerative disease" used herein refers to a progressive disease, which is caused by gradual degeneration of neuron cells in the central nerve system because of accumulation of abnormal protein aggregates, leading to dysfunctions and cell death. Among various RBP proteinopathy diseases, in particular, a disease caused by abnormal accumulation or abnormal aggregation of TDP-43 is referred to as TDP-43 proteinopathy. MSP proteinopathy is a type of TDP-43 proteinopathy. Specific examples of RBP proteinopathy include amyotrophic lateral sclerosis (ALS), such as sporadic ALS and familial ALS, frontotemporal lobar degeneration (FTLD), Alzheimer's disease, Lewy body dementia, Huntington's disease, Parkinson's disease, argyrophilic grain dementia (grain disease), Perry syndrome, progressive supranuclear palsy, corticobasal degeneration, and Pick's disease. The nucleic acid of the present invention can also be used for prevention or treatment of neurodegenerative diseases associated with dementia. The nucleic acid of the present invention is particularly useful for prevention or treatment of ALS and FTLD.

In the brain tissue samples obtained from patients with sporadic amyotrophic lateral sclerosis (ALS) and some familial ALS cases, TDP-43 C-terminal fragments with molecular weights of 25 to 35 kDa are observed, and such fragments are known to be associated with pathological conditions. Such C-terminal fragments can aggregate as with the full-length TDP-43, the C-terminal fragments aggregate together with the full-length TDP-43, and the resultant would exert higher toxicity. The nucleic acid of the present invention inhibits aggregation and toxicity of the C-terminal fragments in addition to the full-length TDP-43 in the brain tissue, so that the nucleic acid would also exert therapeutic effects on sporadic ALS.

The nucleic acid of the present invention can be used for prevention or treatment of the neurodegenerative diseases described above. In addition, the nucleic acid of the present invention can suppress progression and prevent recurrence of the neurodegenerative diseases described above. In the present invention, progression suppression and recurrence prevention of the neurodegenerative diseases are within the scope of prevention or treatment of neurodegenerative diseases. In addition, the nucleic acid of the present invention can be used for suppression or inhibition of abnormal accumulation of TDP-43 in the cells and aggregation of TDP-43 in the cytoplasm.

The nucleic acid of the present invention in the form of an agent for prevention or treatment of neurodegenerative diseases can be administered through a parental route, such as the intrathecal, intravenous, intramuscular, hypodermic, intraperitoneal, or non-enteral route, or through an oral route, with a parental route being preferable and intrathecal injection being more preferable. Examples of dosage forms include injection preparations, tablets, capsules, vulcanizing agents, syrup pharmaceuticals, emulsions, and sprays. The nucleic acid of the present invention is mixed with a common pharmaceutical carrier and used in that state. Examples of carriers include, but are not limited to, physiological saline, phosphate-buffered physiological saline, a glucose solution, and buffered physiologic saline. Also, saccharides, such as xylose, trehalose, and fructose, sugar alcohols, such as mannitol, xylitol, and sorbitol, buffers, such as phosphate buffer, citrate buffer, and glutamate buffer, surfactants, such as fatty acid ester, and the like can be used as additives. When mixed with a carrier, nucleic acid content may be adequately selected and may vary depending on a type of pharmaceutical preparation. Such content is preferably 0.001% to 1% by weight. While the amount to be administered is determined in accordance with age, sex, body weight, symptoms, a purpose of treatment, and other conditions of the patient, in general, 0.0001 to 1 mg can be administered to an adult patient through a parental route once or several separate instances a day. Such amount of administration can adequately be increased or decreased depending on a disease type, age, body weight, and symptoms of the patient, and other conditions.

### [Examples]

The present invention is described in greater detail with reference to the following examples, although the present invention is not limited to these examples.

### [Example 1] Searching for a sequence that inhibits TDP-43 aggregation

From among synthetic nucleic acids comprising various TDP-43-binding sequences, sequences exhibiting the highest capacity for aggregation inhibition were searched for.

On the basis of the capacity for aggregation achieved by vigorous agitation of a TDP-43-containing solution (Huang et al., 2013, PLoS One. 8, e64002), the inhibitory effects of the synthetic nucleic acids on TDP-43 aggregation were evaluated. In Example 1, endogenous TDP-43 contained in the brain extract was used. The corpus striatum of the mouse (C57BL/6J) was homogenized in a buffer (50 mM HEPES, pH 7.4, 150 mM NaCl, 10% glycerol, 1% triton X-100, 5 mM MgCl₂, 1 mM EGTA, protease inhibitors, and phosphatase inhibitors), the resultant was centrifuged at 4°C and 20,000× g for 30 minutes, and the supernatant was then recovered. The brain extract was prepared by adjusting the supernatant to 3 mg/ml.

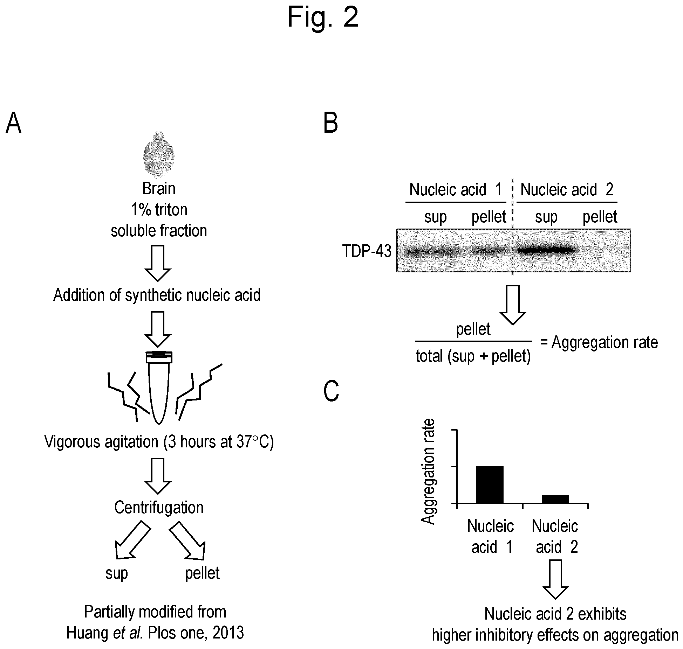

Subsequently, the candidate synthetic nucleic acid was added to the brain extract to the final concentration of 0.1 µM, the resultant was agitated at 37°C and 1,800 rpm for 3 hours, and centrifugation was performed at 4°C and 16,000× g for 30 minutes to separate the supernatant from the precipitate. TDP-43 contained in each fraction was detected via Western blotting, and the aggregation rate (i.e., the proportion of TDP-43 in the precipitate) was calculated. Fig. 2 shows the method for evaluation. Fig. 2A shows the protocols of the method, Fig. 2B shows the method for calculating the aggregation rate, and Fig. 2C shows the method for evaluating the results. The density of the band detected via Western blotting was measured using a densitometer, and the results were quantified to calculate the aggregation rate. As a TDP-43 antibody, a polyclonal antibody (12892-1-AP; Proteintech, Rosemont, IL) was used.

Synthetic nucleic acids were purchased from GeneDesign Inc. or Hokkaido System Science Co., Ltd.

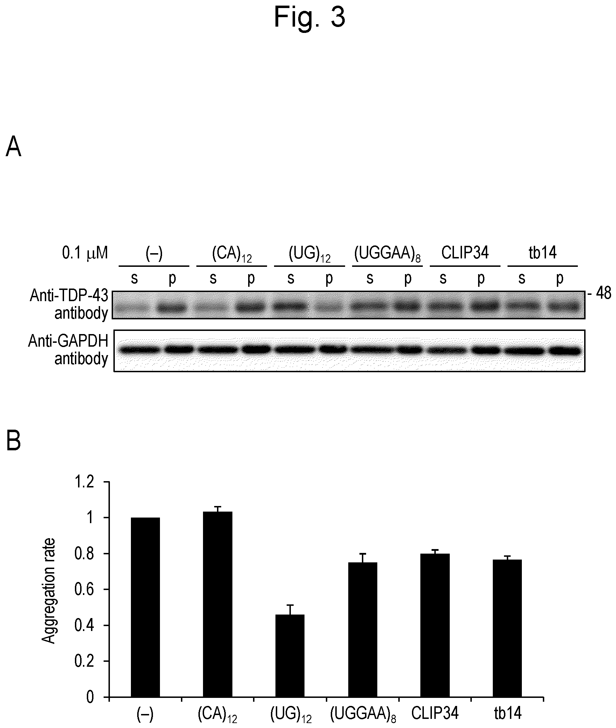

The inhibitory effects of known TDP-43-binding RNAs (24- to 48-mers) on TDP-43 aggregation were examined. Fig. 3 shows the results of examination. In Fig. 3, the symbol (-) indicates the absence of nucleic acids. Candidate synthetic nucleic acids were (CA)₁₂ that would not bind to TDP-43 (a negative control), the (UG)₁₂ sequence, (UGGAA)₈, CLIP34, and tb14. (UGGAA)₈, CLIP34, and tb14 are the sequences that have been reported to bind to TDP-43. (UGGAA)ₙ is a sequence of an abnormally elongated region observed in the causal gene of spinocerebellar ataxia type 31 (Ishiguro et al., 2017, Neuron. 94, 108-24), CLIP34 is a sequence of the TDP-43-binding region in 3'-UTR of messenger RNA of the TDP-43 (GAGAGAGCGCGUGCAGAGACUUGGUGGUGCAUAA: SEQ ID NO: 1; Ayala et al.,

2011), and tb14 is a TDP-43-binding sequence identified by SELEX (GAAGUGGGAUGUGGGAAGAGGGAUGUUGUG: SEQ ID NO: 2; Ishiguro et al., 2016, Genes Cells, 21, 466-81).

RNA was added to the final concentration of 0.1 µM. Fig. 3A shows the results of Western blotting, and Fig. 3B shows the results of quantification of the density of the band detected via Western blotting using a densitometer. In Fig. 3A, "s" indicates a supernatant, and "p" indicates a precipitate. Fig. 3B shows the aggregation rate of each RNA by designating the aggregation rate of (-) (in the absence of nucleic acids) as 1. The results demonstrate that (UG)₁₂ exhibits the highest inhibitory effects on aggregation.

### [Example 2] Searching for chemical modifications that enhance effects of RNA of the (UG)₁₂ sequence

Chemical modifications that would enhance the inhibitory effects of the (UG)₁₂ sequence obtained in Example 1 on aggregation were searched for.

The inhibitory effects of synthetic nucleic acids A to D each comprising the (UG)₁₂ sequence as a backbone on aggregation were examined. As a negative control, synthetic nucleic acids comprising (CA)₁₂ as a backbone were examined at the same time.

The chemical modifications examined were substitution of RNA with DNA (a DNA is indicated by a lower-case alphabet), 2'-O-methylation (2'OMe), and a locked nucleic acid (LNA). Specifically, nucleic acids consisting of the nucleotide sequences shown below were used. "(Me)" indicates 2'-O-methylation, and "(L)" indicates LNA.
A: (U(Me)G(Me))₁₂
B: (tG(L))₁₂
C: (UG(L))₁₂
D: (U(Me)G(L))₁₂

The four types of combinations above were examined.

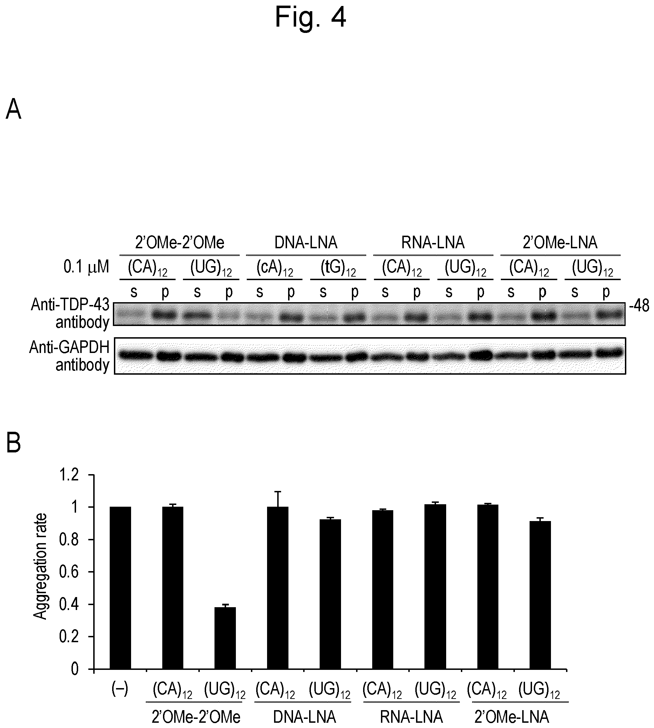

The results are shown in Fig. 4. Fig. 4A shows the results of Western blotting, and Fig. 4B shows the results of quantification of the density of the band detected via Western blotting using a densitometer. The aggregation rate was calculated based on the density of the band of each fraction detected via Western blotting. As a result, the nucleic acids in which both the uracil nucleotide and the guanine nucleotide had been 2'-O-Me-modified were found to exert the highest inhibitory effects (Fig. 4B).

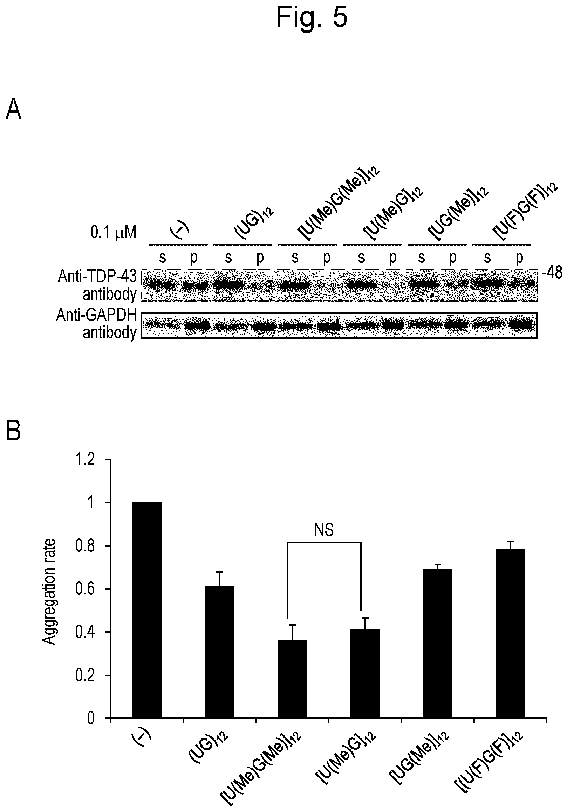

On the basis of the results, modification of the 2'-O atom was further analyzed. The results are shown in Fig. 5. Fig. 5A shows the results of Western blotting, and Fig. 5B shows the results of quantification of the density of the band detected via Western blotting using a densitometer. At the outset, the effects of (UG)₁₂ were compared with the results of (U(Me)G(Me))₁₂. As a result, the inhibitory effects of (UG)₁₂ on aggregation were found to enhance to a significant extent (p < 0.05) via 2' OMe modification. In contrast, the nucleic acids in which both the uracil nucleotide and the guanine nucleotide were subjected to 2'-fluoridation (2'F), which is the same 2'-O atom modification ([U(F)G(F)] ₁₂), did not show the enhanced inhibitory effects on aggregation. Synthetic nucleic acids in which either the uracil nucleotide or the guanine nucleotide had been 2'-O-Me-modified were then examined. The results demonstrate that the effects equivalent to those of (U(Me)G) and (U(Me)G(Me))₁₂ would be achieved by selective 2'-O-Me-modification of the uracil nucleotide (no significant differences), and the inhibitory effects of (UG(Me)) and (UG)₁₂ on aggregation would not be enhanced upon selective 2'-O-Me-modification of the guanine nucleotide. On the basis of the results indicated above and the results shown in Fig. 4, the inhibitory effects on aggregation would not be achieved with a nucleic acid with the uracil nucleotide being 2'-O-Me-modified but the guanine nucleotide being LNA. Accordingly, the nucleic acid that would inhibit TDP-43 aggregation (i.e., (UG)₁₂) was found to enhance its effects with the uracil nucleotide being 2'-O-Me-modified and the guanine nucleotide being RNA or 2'-O-Me-modified.

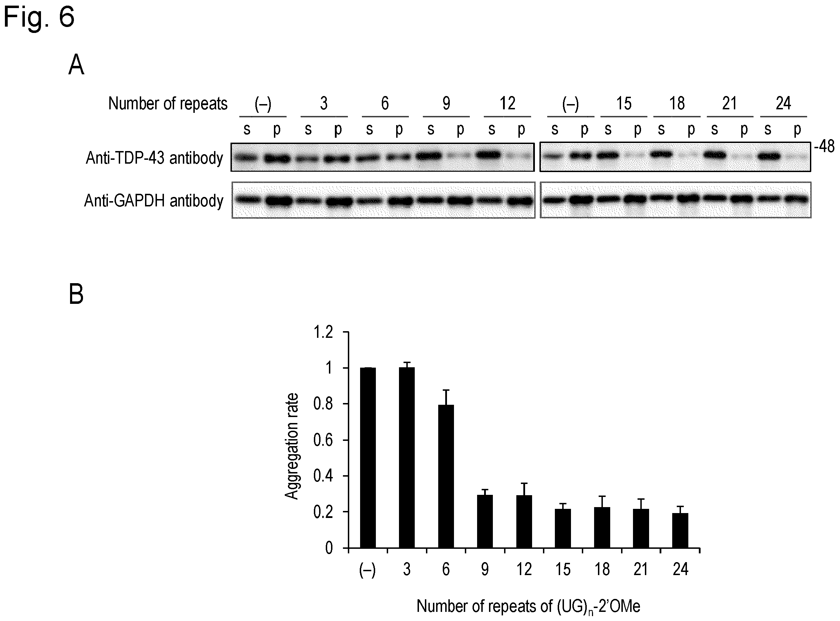

Subsequently, the number of repeats of (U(Me)G(Me))n was examined (n = 3 to 24). The results are shown in Fig. 6. As shown in Fig. 6, the inhibitory effects on aggregation were observed when "n" was 9 or more; that is, the total number of nucleotides was 18 or more.

### [Example 3] Effects of 2'-O-Me-modification on sequences other than (UG)₁₂

Whether or not 2'-O-Me-modification that was found to be a chemical modification that would enhance the inhibitory effects on aggregation in Example 2 would be effective on sequences other than (UG)₁₂ was examined.

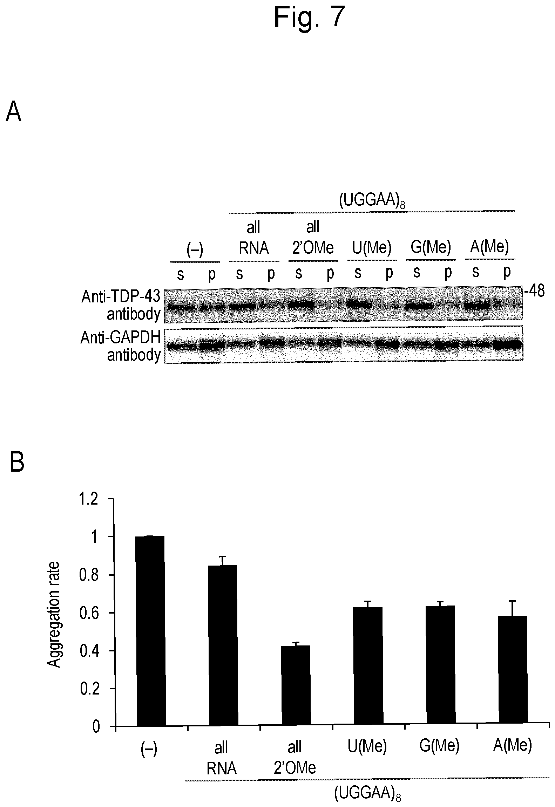

In the past, RNA of the (UGGAA)ₙ sequence was reported to bind to TDP-43 (Ishiguro et al., 2017; WO 2016/088797). On the basis of this sequence, whether or not 2'-O-Me-modification would enhance the inhibitory effects on aggregation in (UGGAA)₈ was examined. The effects of (UGGAA)₈ in which all the nucleotides had been 2'-O-Me-modified were examined by the method described in Example 1. The results are shown in Fig. 7. As shown in Fig. 7, the inhibitory effects were enhanced to a significant extent compared with (UGGAA)₈ without chemical modification (with all nucleotides being RNAs), and 2'-O-Me-modification was found to enhance the inhibitory effects on aggregation in the (UGGGAA)₈ sequence. While the highest inhibitory effects on aggregation were achieved when all the nucleotides had been 2'-O-Me-modified, the inhibitory effects higher than those of (UGGAA)₈ (with all nucleotides being RNAs) were achieved when the first U in the UGGGAA sequence was 2'-O-Me-modified.

### [Example 4] Effectiveness of (U(Me)G(Me))₁₂ in vivo

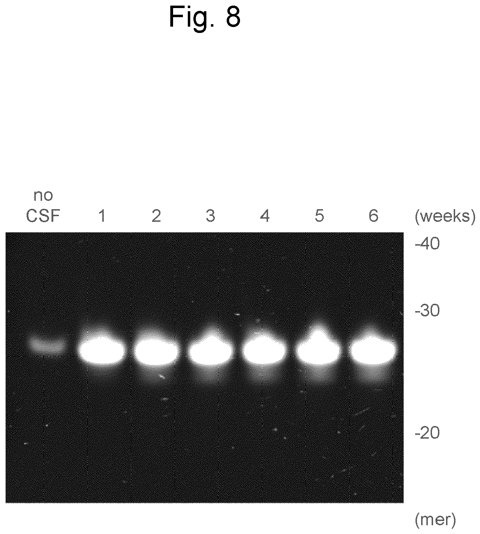

In order to evaluate the effectiveness of (U(Me)G(Me))₁₂, which was found to exhibit satisfactory effects via *in vitro* evaluation, in model animals, stability thereof in the cerebrospinal fluid (CSF) was examined. To 40 µl of commercially available human cerebrospinal fluid, 5 µl of 20 µg/µl nucleic acid was added, and the resultant was allowed to stand at 37°C. (U(Me)G(Me))₁₂ was collected every week up to the 6th week, subjected to acrylamide gel electrophoresis, visualized, and then detected. (U(Me)G(Me))₁₂ was not substantially degraded 6 weeks after it was allowed to stand, and stability thereof in the cerebrospinal fluid was confirmed (Fig. 8).

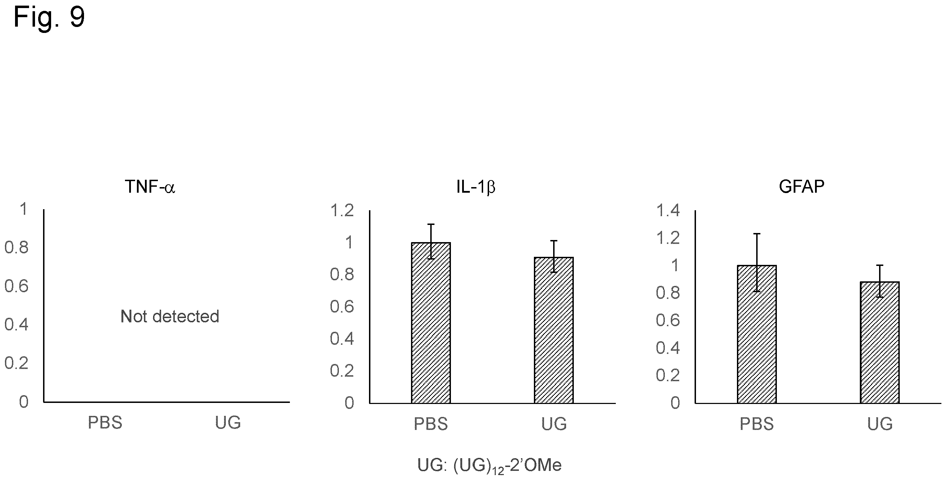

Safety of (U(Me)G(Me))₁₂ and ((UG)₁₂-2'OMe) was then examined. Under anesthesia, 5 µl of 20 µg/µl nucleic acid was administered intraventricularly to 5-week-old wild-type mice. With reference to the 11 items shown in Table 1, mouse behaviors were observed to examine the presence or absence of acute toxicity (a higher score is obtained in case of higher toxicity). The items shown in Table 1 were prepared with reference to US 2016/0304871 A1 and WO 2016/127000 A1. As a result, no abnormal behaviors were observed in mice 30 minutes and 3 hours later (the score: 0). Toxicity was also evaluated based on nerve cell death and detection of inflammation. Two weeks after intraventricular nucleic acid administration, RNA was extracted from the brain tissue in the vicinity of the site of administration, and expression levels of GFAP (the gliosis index) and TNF-α and IL-1β (the inflammation indices) were evaluated via quantitative real-time PCR. No elevation was observed in any indices, compared with the control group to which PBS had been administered. It was thus considered that evaluation using model animals could be safely performed (Fig. 9).

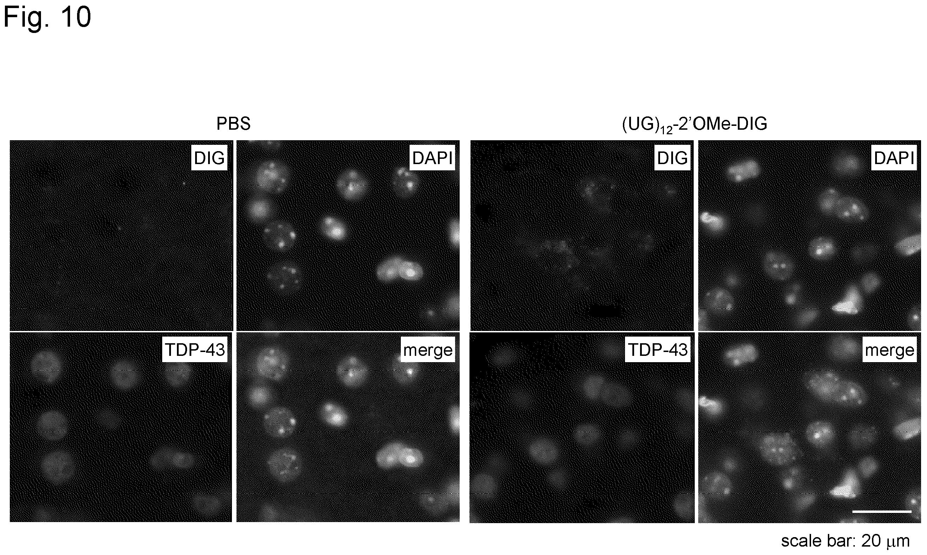

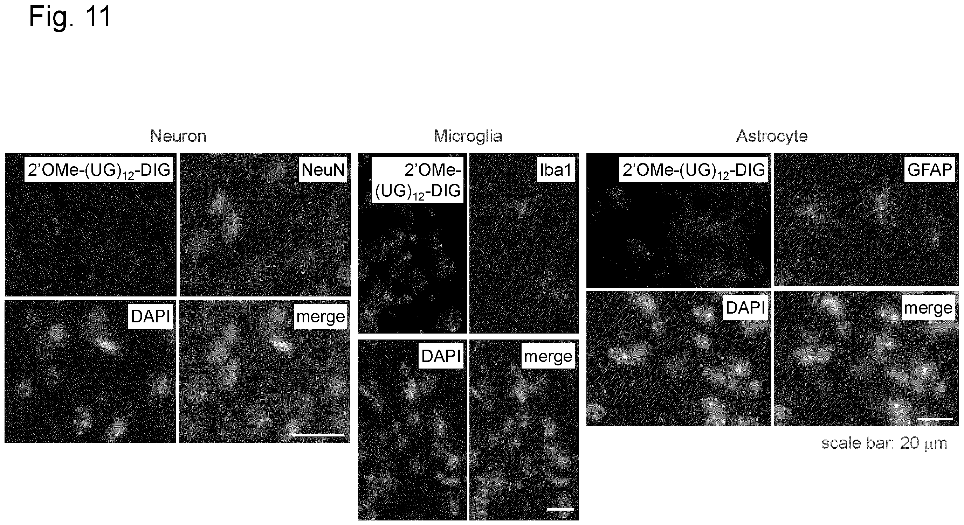

Subsequently, the intracerebral distribution and the intracellular localization of the (U(Me)G(Me))₁₂ administered were examined. (U(Me)G(Me))₁₂ ((UG)₁₂-2'OMe-DIG) labeled with digoxigenin (DIG) at the 3'-terminus were administered intraventricularly for detection via fluorescent staining and perfusion-fixed 3 days later to prepare brain sections. The brain sections were fluorescent-stained using DIG antibody and TDP-43 antibody (Fig. 10). Separately, the brain sections were fluorescent-stained using DIG antibody, NeuN antibody (nerve cell marker), Iba1 antibody (microglia marker), and GFAP antibody (astrocyte marker) (Fig. 11). PBS was administered as a control sample. Nuclei were stained with DAPI. As a result, DIG-positive cells were observed in the right brain in addition to the left brain to which (U(Me)G(Me))₁₂ had been administered, and (U(Me)G(Me))₁₂ was thus found to be extensively distributed in the brain (Fig. 10). In addition, DIG signals were not substantially co-localized with microglia cells or astrocytes but many DIG signals were observed in NeuN-positive cells. Since DIG signals are not co-localized with DAPI, (U(Me)G(Me))₁₂ was found to be mostly localized in the neuron cytoplasm (Fig. 11).

**[Table 1] acute tolerability score (0 if present; 1 if abnormal)**

| | | |
|---|---|---|
| 1. | Alert, bright and responsive | |
| 2. | Standing or hunching without stimuli | |
| 3. | Shows movements without stimuli | |
| 4. | Shows forward movement after being lifted up | |
| 5. | Shows any movements after being lifted up | |
| 6. | Responds to tail pinch | |
| 7. | Regular breathing | |
| 8. | No hyperactivity | |
| 9. | No motor dysfunction and ataxia | |
| 10. | Normal posture | |
| 11. | No tremors/convulsions | |

| | | |
|---|---|---|
| score: 0-11 | | |

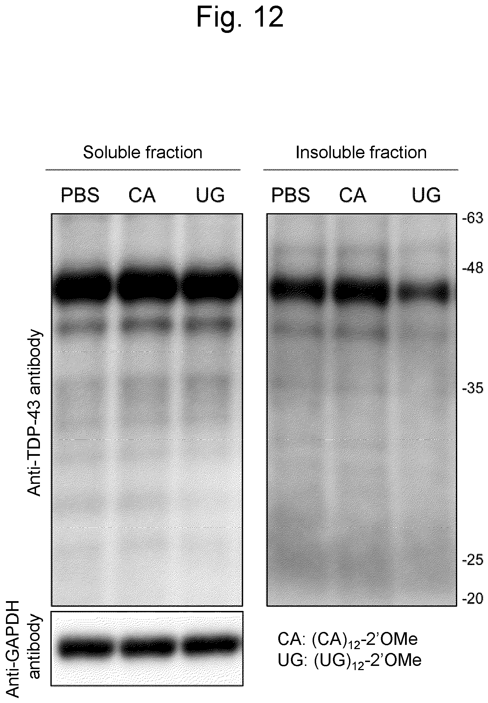

On the basis of the results attained above, it was considered that effectiveness evaluation could be performed using animal models. Thus, (U(Me)G(Me))₁₂ and ((UG)₁₂-2'OMe) were administered intraventricularly, and effects thereof *in vivo* were examined. As animal models, transgenic mice overexpressing human TDP-43 with a defective nuclear transfer signal (hTDP-43-ΔNLS) in a neuron-specific manner were purchased from Jackson Laboratory. As a result of removal of doxycycline (DOX) from feeds, the mice are capable of inducing hTDP-43-ΔNLS expression (tet-off-inducible), and, as a result of overexpression, TDP-43 aggregation in the neuron cytoplasm, nerve cell death, and abnormality in motor functions are observed in such animal models. After mice were raised to 5-week-old by feeding them with DOC-containing feeds, 100 µg of (U(Me)G(Me))₁₂ was administered intraventricularly under anesthesia. At the same time, they were fed with DOX-free feeds to induce hTDP-43-ΔNLS expression. The left half of the motor area was collected 2 weeks after administration to detect the amount of TDP-43 in a fraction containing TDP-43 aggregates (a fraction insoluble in RIPA) via Western blotting. As a control, ((CA)₁₂-2'OMe) was used. As a result, it was found that TDP-43 aggregation was inhibited by (U(Me)G(Me))₁₂ administration (Fig. 12).

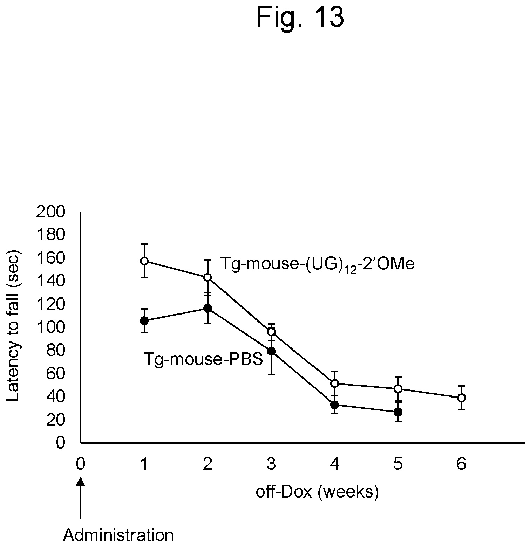

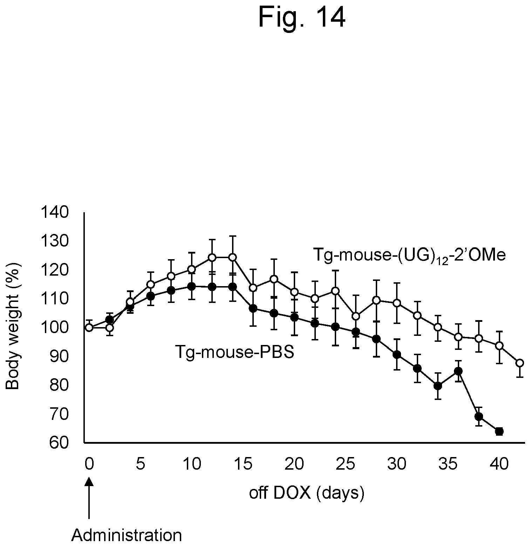

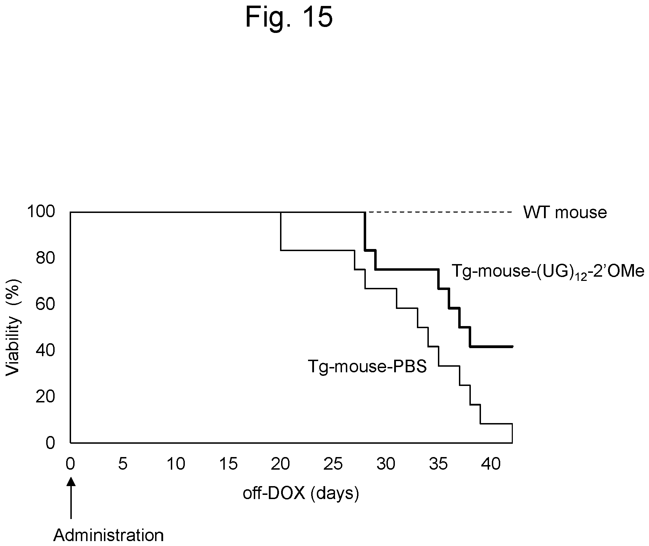

Subsequently, the rotarod test assessing motor coordination was performed. As a result, the latency to fall (seconds) was found to improve upon administration of (U(Me)G(Me))₁₂ ((UG)₁₂-2'OMe), and effects of alleviating motility disturbance were observed (Fig. 13). Also, changes in mouse body weight were measured, and effects of deterring body weight loss caused by hTDP-43-ΔNLS overexpression were observed in the group to which (U(Me)G(Me))₁₂ had been administered (Tg-mouse-(UG)₁₂-2'OMe), compared with the control group to which PBS had been administered (Tg-mouse-PBS) (Fig. 14). All the mice of the group to which PBS had been administered died up to 42 days after the initiation of overexpression. On the same day, in contrast, 42% of the mice survived in the group to which (U(Me)G(Me))₁₂ ((UG)₁₂-2'OMe) had been administered (Fig. 15). On the basis of the results, (U(Me)G(Me))₁₂ was found to be effective for improvement of pathological conditions associated with TDP-43 overexpression and it was found to be useful as an agent for treatment of ALS.

### [Industrial Applicability]

The nucleic acid of the present invention can be used as an agent for prevention or treatment of neurodegenerative diseases caused by abnormal expression of RNA-binding proteins.

All publications, patents, and patent applications cited herein are incorporated herein by reference in their entirety.

## Claims

1. A natural or artificial nucleic acid that binds to an RNA recognition motif (RRM).

2. The nucleic acid according to claim 1, which binds to an RNA recognition motif (RRM) of TDP-43.

3. The nucleic acid according to claim 1 or 2, which comprises a repeat RNA consisting of nucleotides represented by the formula: U(Mod)GX₁X₂X₃...Xₘ)ₙ, wherein U(Mod) represents a U derivative having an oxygen atom at the ribose 2' position; X₁, X₂, X₃, ...Xₘ may or may not be present, each independently represent A, C, G, or U in the presence thereof, and may be the same or different; m is 1 to 10; G, X₁, X₂, X₃, ...Xₘ may or may not each independently represent a derivative having an oxygen atom at the 2' position; and n is 4 to 15.

4. The nucleic acid according to claim 3, wherein U(Mod) represents 2'-O-methylated or 2'-O-methoxyethylated (MOE) U.

5. The nucleic acid according to claim 4, which comprises a repeat RNA consisting of nucleotides represented by the formula: U(Me)GX₁X₂X₃...Xₘ)ₙ, wherein U(Me) is 2'-O-Me-modified; X₁, X_{2,} X₃, ...Xₘ may or may not be present, each independently represent A, C, G, or U in the presence thereof, and may be the same or different; m is 1 to 10; G, X₁, X_{2,} X₃, ...Xₘ may or may not be each independently 2'-O-Me-modified; and n is 4 to 15.

6. The nucleic acid according to claim 5, which comprises a repeat RNA consisting of nucleotides represented by the formula: (U(Me)G) ₙ or (U(Me)G(Me)) ₙ, wherein U(Me) and G(Me) are each independently 2'-O-Me-modified; and n is 7 to 30.

7. The nucleic acid according to claim 5, which comprises a repeat RNA consisting of nucleotides represented by the formula: (U(Me)G)₁ or (U(Me)G(Me))₁₂, wherein U(Me) and G(Me) are each independently 2'-O-Me-modified.

8. The nucleic acid according to claim 5, which comprises a repeat RNA consisting of nucleotides represented by the formula: (U(Me)G(Me)G(Me)A (Me)A (Me))₅₋₁₀, wherein U(Me), G(Me), and A (Me) are 2'-O-Me-modified.

9. The nucleic acid according to claim 4, which comprises a repeat RNA consisting of nucleotides represented by the formula: (U(MOE)GX₁X₂X₃ Xₘ)ₙ, wherein U(MOE) is 2'-O-methoxyethylated; X₁^{,} X_{2,} X₃, ...Xₘ may or may not be present, each independently represent A, C, G, or U in the presence thereof, and may be the same or different; m is 1 to 10; G, X₁, X₂, X₃, ...Xₘ may or may not be each independently 2'-O-methoxyethylated; and n is 4 to 15.

10. The nucleic acid according to claim 9, which comprises a repeat RNA consisting of nucleotides represented by the formula: (U(MOE)G)ₙ or (U(MOE)G(MOE))ₙ, wherein U(MOE) and G(MOE) are 2'-O-methoxyethylated; and n is 7 to 30.

11. The nucleic acid according to claim 9, which comprises a repeat RNA consisting of nucleotides represented by the formula: (U(MOE)G)₁₂ or (U(MOE)G(MOE))₁₂, wherein U(MOE) and G(MOE) are 2'-O-methoxyethylated (MOE).

12. The nucleic acid according to claim 9, which comprises a repeat RNA consisting of nucleotides represented by the formula: (U(MOE)G(MOE)G(MOE)A (MOE)A (MOE))₅₋₁₀, wherein U(MOE), G(MOE), and A (MOE) are 2'-O-methoxyethylated.

13. The nucleic acid according to any one of claims 3 to 12, wherein at least 1 RNA in the repeat RNA is substituted with DNA.

14. The nucleic acid according to any one of claims 1 to 4, which comprises at least 5 dinucleotide units represented by the formula: (U(Mod)G) or (U(Mod)G(Mod)), wherein U(Mod) and G(Mod) each independently represent 2'-O-methylated or 2'-O-methoxyethylated U and G.

15. The nucleic acid according to any one of claims 1 to 14, wherein the total number of nucleotides is 10 to 50.

16. A pharmaceutical composition comprising, as an active ingredient, the nucleic acid according to any one of claims 1 to 15.

17. An agent for prevention or treatment of a neurodegenerative disease induced by abnormal accumulation of TDP-43 in cells comprising, as an active ingredient, the nucleic acid according to any one of claims 1 to 15.

18. The agent for prevention or treatment of a neurodegenerative disease according to claim 17, wherein the nucleic acid suppresses abnormal accumulation of TDP-43 in cells.

19. The agent for prevention or treatment of a neurodegenerative disease according to claim 17 or 18, wherein the neurodegenerative disease is TDP-43 proteinopathy.

20. The agent for prevention or treatment of a neurodegenerative disease according to any one of claims 17 to 19, wherein the neurodegenerative disease is selected from the group consisting of amyotrophic lateral sclerosis (ALS), which is sporadic ALS or familial ALS, frontotemporal lobar degeneration (FTLD), Alzheimer's disease, Lewy body dementia, Huntington's disease, Parkinson's disease, argyrophilic grain dementia (grain disease), Perry syndrome, progressive supranuclear palsy, corticobasal degeneration, and Pick's disease.

21. The agent for prevention or treatment of a neurodegenerative disease according to claim 20, wherein the neurodegenerative disease is amyotrophic lateral sclerosis (ALS), which is sporadic ALS or familial ALS, or frontotemporal lobar degeneration (FTLD).
